# EUROPÄISCHE PATENTSCHRIFT

(11) **EP 1 602 640 B1**
(45) Veröffentlichungstag und Bekanntmachung des Hinweises auf die Patenterteilung: **20.08.2008**
(21) Anmeldenummer: 05010842.2
(22) Anmeldetag: 19.05.2005
(51) Int. Cl.: C07C 209/86

(54) **Verfahren zur destillativen Trennung von wässrigen Aminlösungen**
Process for separation of aqueous amine solutions by distillation
Procédé pour la séparation par distillation de solutions aqueuses d'amines

(30) Priorität: 01.06.2004 DE 102004026626
(43) Veröffentlichungstag der Anmeldung: 07.12.2005
(73) Patentinhaber: Bayer MaterialScience AG, 51368 Leverkusen (DE)
(72) Erfinder: Steffens, Friedhelm, 51373 Leverkusen (DE); Buse, Rainer, Dr., 51061 Köln (DE); Brady, Bill, 40489 Düsseldorf (DE)

(56) Entgegenhaltungen:
- EP-A- 0 236 839
- EP-A- 0 794 170
- EP-A- 1 312 600
- WO-A-03/042159

## Beschreibung

Die Erfindung betrifft ein energiegünstiges Verfahren zur destillativen Aufarbeitung wässriger Aminlösungen, die bei der katalytischen Hydrierung von Nitroaromaten anfallen. Bei diesem Verfahren kann sowohl das Amin von Wasser befreit als auch das Wasser amin- und leichtsiederfrei erhalten als auch die konzentrierten Leichtsieder gewonnen werden.

Es ist bekannt (DE-A-1 542 544, DE-A-1 947 851, DE-A-2 106 644, DE-A-2 135 154, DE-A-2 214 056, DE-A-2 456 308, BE-PS-631 964, BE-PS-661 047, BE-PS-661 946, FR-PS 1 359 438 oder GB-PS 768 111), dass man aromatische Diamine durch katalytische Hydrierung der entsprechenden aromatischen Dinitroverbindungen herstellen kann. Die Hydrierung kann unter Mitverwendung von Lösungsmitteln wie beispielsweise niedrig siedenden Alkoholen wie Methanol, Ethanol oder Isopropanol aber auch ohne solche Fremdlösungsmittel erfolgen. Die Hydrierung kann mit Hilfe von im Reaktionsgemisch dispergierten Katalysatoren durchgeführt werden, die dann durch Sedimentation oder Filtration abgetrennt und gegebenenfalls in den Prozess zurückgeführt werden.

Bislang erfolgte die Aufarbeitung des Reaktionsgemisches dergestalt, dass man ein nach Abtrennung des gegebenenfalls mitverwendeten Hilfslösungsmittels vorliegendes Gemisch aus aromatischen Diaminen und Reaktionswasser zunächst kontinuierlich unter Normaldruck in einer Destillationskolonne von Wasser befreit und anschließend das als Destillationsrückstand anfallende Diamin gegebenenfalls in weiteren Verfahrensschritten von noch anhaftendem Wasser und von gegebenenfalls noch vorliegenden organischen Verunreinigungen befreit. Bei dieser Arbeitsweise entstehen als Destillate stets Gemische von Wasser mit wasserdampfflüchtigen organischen Nebenprodukten, wie sie bei der Hydrierung der Dinitroaromaten anfallen. Es handelt sich bei diesen Nebenprodukten beispielsweise um aromatische oder cycloaliphatische Monoamine und/oder um cycloaliphatische Alkohole, d. h. beispielsweise im Falle der Herstellung von Diaminotoluol um Toluidine, Perhydrotoluidine und/oder Methylcyclohexanole.

Diese wasserdampfflüchtigen Nebenprodukte bewirken, dass das über Kopf abdestillierte Wasser stark mit diesen Verbindungen belastet ist. In EP 0 236 839 B1 ist ein Verfahren zur destillativen Aufarbeitung von derartigen wässrigen Aminlösungen beschrieben, bei welchem ein weit weniger mit organischen Verunreinigungen belastetes Abwasser anfällt. Dazu wird das Gemisch in einer Destillationskolonne mit Seitenentnahme aufgetrennt. Die Brüden der Destillationskolonne werden kondensiert, die hierbei anfallende flüssige Phase über eine Phasentrennapparatur geführt, in welcher dem Brüdenkondensat wasserdampfflüchtige organische Nebenprodukte als organische Phase entnommen werden. Die wässrige Phase wird auf den Kopf der Destillationskolonne zurückführt. Das weitgehend von wasserdampfflüchtigen organischen Verunreinigungen befreite Wasser wird über einen Seitenstrom entnommen. Die von Wasser und wasserdampfflüchtigen Verunreinigungen befreiten Diamine fallen dabei als Sumpfprodukt an. All diesen Verfahren ist jedoch der hohe Energieeinsatz gemeinsam: pro kg abzutrennendem Wasser müssen 1,2 bis 2 kg Heizdampf eingesetzt werden.

Die Aufgabe der vorliegenden Erfindung besteht daher darin, ein einfaches und wirtschaftliches Verfahren zur destillativen Auftrennung der wässrigen Aminlösungen, die bei der katalytischen Hydrierung der entsprechenden Nitroaromaten anfallen, zur Verfügung zu stellen, das unter geringem Energieeinsatz betrieben werden kann und bei dem das abgetrennte Wasser im Wesentlichen frei von wasserdampfflüchtigen organischen Verunreinigungen erhalten werden kann.

Die Erfindung betrifft ein Verfahren zur destillativen Trennung von wässrigen Aminlösungen, die bei der Hydrierung von Nitroaromaten anfallen, dadurch gekennzeichnet, dass
a) die Destillation in mindestens zwei in Serie verschalteten Destillationskolonnen durchgeführt wird, die mit unterschiedlichen Drücken betrieben werden, wobei wenigstens eine Destillationskolonne bei absoluten Drücken am Kopf von 2 bis 20 bar betrieben wird und mindestens eine Destillationskolonne bei absoluten Drücken am Kopf von 0,1 bis 10 bar betrieben wird, und
b) die aus der mit höheren Drücken betriebenen Destillationskolonne austretenden Brüden zumindest teilweise kondensiert werden und die dabei freiwerdende Wärme zur Beheizung des Sumpfs der bei den niedrigeren Drücken betriebenen Destillationskolonne verwendet wird, und
c) die Aminlösung in die erste der mindestens zwei in Serie verschalteten Destillationskolonnen geführt wird, und der Sumpf der ersten Destillationskolonne teilweise abgezogen und in die zweite Destillationskolonne eingespeist wird, und
d) das gereinigte Amin als Sumpfprodukt der letzten Destillationskolonne abgezogen wird.

Die Erfindung betrifft insbesondere ein Verfahren zur destillativen Trennung von wässrigen Aminlösungen, die bei der Hydrierung von Nitroaromaten anfallen, dadurch gekennzeichnet, dass
a) die Destillation in mindestens zwei in Serie verschalteten Destillationskolonnen durchgeführt wird, die mit unterschiedlichen Drücken betrieben werden, wobei wenigstens eine Destillationskolonne bei absoluten Drücken am Kopf von 2 bis 20 bar betrieben wird und mindestens eine Destillationskolonne bei absoluten Drücken am Kopf von 0,1 bis 10 bar betrieben wird, und
b) die aus der mit höheren Drücken betriebenen Destillationskolonne austretenden Brüden zumindest teilweise kondensiert werden und die dabei freiwerdende Wärme zur Beheizung des Sumpfs der bei den niedrigeren Drücken betriebenen Destillationskolonne verwendet wird, und
c) die Aminlösung in die erste der mindestens zwei in Serie verschalteten Destillationskolonnen geführt wird, und der Sumpf der ersten Destillationskolonne teilweise abgezogen und in die zweite Destillationskolonne eingespeist wird, und
d) das gereinigte Amin als Sumpfprodukt der letzten Destillationskolonne abgezogen wird, und
e) die Brüden aus der mit den geringeren Drücken betriebenen Destillationskolonne kondensiert werden, die wasserdampfflüchtigen Leichtsieder durch Phasentrennung daraus abgetrennt werden, und die verbleibenden wässrigen kondensierten Brüden als Rücklauf in die mit den geringeren Drücken betrieben Destillationskolonne zurückgeführt werden, und
f) das Brüdenkondensat aus der mit den höheren Drücken betriebenen Destillationskolonne teilweise auf den Kopf der mit den niedrigeren Drücken betriebenen Destillationskolonne aufgegeben wird und teilweise als Rücklauf auf den Kopf der mit den höheren Drücken betriebenen Kolonne aufgegeben wird, und
g) Wasser als Seitenstrom aus der mit den niedrigeren Drücken betriebenen Destillationskolonne abgezogen wird.

Bevorzugt wird die Destillation in zwei in Serie verschalteten Destillationskolonnen durchgeführt. Es ist aber grundsätzlich auch möglich, mehr als zwei Destillationskolonnen einzusetzen die in Richtung der seriellen Verschaltung mit fallenden oder steigenden Drücken betrieben werden, wobei dann die Brüden aus der Kolonne mit dem nächsthöheren Druck jeweils für die Beheizung des Sumpfs der benachbarten Kolonne mit dem nächstniedrigeren Druck verwendet werden können. Die Leichtsieder- und Wasserabtrennung findet dabei bevorzugt in der Kolonne statt, die mit dem niedrigsten Druck betrieben wird, weil dann hierbei die Dichtedifferenz maximal ist. Es ist aber auch grundsätzlich möglich, die Leichtsieder- und Wasserabtrennung in der Kolonne mit dem höheren Druck oder aber in beiden Destillationskolonnen vorzunehmen. Das gereinigte Amin wird dann in Schritt d) in der letzten Destillationskolonne, bei insgesamt zwei in Serie verschalteten Destillationskolonnen entsprechend in der zweiten Destillationskolonne, abgezogen.

Durch das erfindungsgemäße Verfahren wird erreicht, dass die wässrigen, nebenprodukthaltigen Aminlösungen unter minimiertem Energieeinsatz gezielt in die drei Bestandteile Amin, Nebenprodukt und reines Wasser aufgetrennt werden. Das Besondere dieses Verfahrens ist, dass diese Stofftrennung in mindestens zwei separaten Kolonnen bei unterschiedlichen Drücken durchgeführt wird, wobei die eine Kolonne die andere beheizt. Hierdurch wird der Energieverbrauch deutlich gesenkt. Am Kopf einer der Kolonne können die wasserdampfflüchtigen Nebenprodukte aufkonzentriert, als Brüden abgezogen und kondensiert und über eine Flüssig-Flüssig-Phasentrennapparatur von organischen Bestandteilen getrennt und ausgeschleust werden.

Bevorzugt dient das erfindungsgemäße Verfahren zur Herstellung von aromatischen Diaminen, insbesondere von Toluylendiamin.

Ausgangsmaterialien für das erfindungsgemäße Verfahren sind insbesondere wässrige Aminlösungen, wie sie bei der Hydrierung von Dinitroaromaten anfallen. Besonders bevorzugt werden beim erfindungsgemäßen Verfahren wässrige Aminlösungen eingesetzt, wie sie bei der Hydrierung von technischen Dinitrotoluolen anfallen, und die von gegebenenfalls bei der Hydrierung mitverwendetem Hilfslösungsmittel, wie den oben genannten einfachen Alkoholen, vorab destillativ befreit worden sind. Bei diesen Lösungen handelt es sich im allgemeinen um ca. 50 - 70 gew.-%ige, vorzugsweise 55 - 65 gew.-%ige Lösungen (bezogen auf das Gewicht der Lösung) von Diaminotoluolen in Wasser, wobei diese Lösungen im allgemeinen bis zu 5 Gew.-% (bezogen auf das Gewicht der Lösung), vorzugsweise 500 - 5000 ppm (Gewicht) an wasserdampfflüchtigen Verunreinigungen der oben beispielhaft genannten Art enthalten. Bei diesen Diaminen handelt es sich z. B. um reines 2,4-Diaminotoiuol oder um dessen technische Gemische mit bis zu 40 Gew.-%, bezogen auf Gesamtgewicht, an 2,6-Diaminotoluol und gegebenenfalls bis zu 5 Gew.-%, bezogen auf Gesamtgemisch, an anderen isomeren Diaminotoluolen, wobei sich die Prozentsätze jeweils zu 100 ergänzen.

Zur Durchführung des erfindungsgemäßen Verfahrens werden bevorzugt zwei Destillationskolonnen, beispielsweise Glockenboden-, Packungs- oder Füllkörperkolonnen seriell hintereinander verschaltet eingesetzt. Vorzugsweise weisen die beiden Kolonnen jeweils 12 bis 50 insbesondere 20 bis 40 theoretische Trennstufen auf. Die Kolonne mit dem niedrigeren Druck wird bevorzugt bei einer Sumpftemperatur von 60 bis 150, vorzugsweise 90 bis 110°C, betrieben. Der absolute Druck am Kopf der Kolonne beträgt 0,1 bis 10 bar, vorzugsweise 0,5 bis 6 bar.

Die Kolonne mit dem höheren Druck wird bevorzugt bei einer Sumpftemperatur von 120 bis 240°C, besonders bevorzugt 180 bis 220°C betrieben. Der absolute Druck am Kopf der Kolonne beträgt 2 bis 20 bar, vorzugsweise 3 bis 6 bar. Die Drücke in den beiden Kolonnen werden bevorzugt so gewählt, dass sich im Wärmetauscher, in dem die Brüden aus der Kolonne mit dem höheren Druck kondensiert werden und gleichzeitig der Sumpf der Kolonne mit dem niedrigeren Druck aufgeheizt wird, mindestens eine Temperaturdifferenz zwischen kondensierender und verdampfender Seite von bevorzugt mindestens 10°C, besonders bevorzugt mindestens 20°C ergibt. Die einzustellenden Betriebsbedingungen in den zwei Kolonnen sind selbstverständlich von der Natur der aufzuarbeitenden Gemische, dem Heizdampfniveau und von der angestrebten Brüdentemperatur der Niederdruckkolonne abhängig. Bei geeigneter Wahl könne diese Brüden z.B. beispielsweise zur Verdampfung von Lösungsmitteln, zur Erwärmung von Produktströmen oder zur Erzeugung von Prozessdampf genutzt werden. Je nach Niveau der Heizdampftemperatur kann die Fremdbeheizung in einem Apparat oder in mehreren erfolgen.

Insbesondere für die Trennung von wässrigen Aminlösungen, die bei der Hydrierung von technischen Dinitrotoluolen anfallen, und die von gegebenenfalls bei der Hydrierung mitverwendetem Hilfslösungsmittel, wie Alkoholen, vorab destillativ befreit worden sind, bieten sich zwei bevorzugte Ausführungsformen des erfindungsgemäßen Verfahrens an. Diese Ausführungsformen sind jedoch nicht auf die Trennung dieser speziellen wässrigen Aminlösungen beschränkt.

In einer ersten Ausführungsform des erfindungsgemäßen Verfahrens wird die erste der mindestens zwei seriell verschalteten Destillationskolonnen mit einem geringeren absoluten Druck im Bereich von 0,1 bis 10 bar betrieben. Die zweite Destillationskolonne (oder im Falle von insgesamt mehr als zwei Destillationskolonnen eine der nachfolgenden Destillationskolonnen) wird also bei höheren Drücken betrieben als die erste Destillationskolonne. Die zu trennende wässrige Aminlösung wird oberhalb des Sumpfverdampfers, vorzugsweise zwischen der zweiten und der achten theoretischen Stufe (vom Sumpf der Kolonne), in die erste Kolonne eingespeist. Der Sumpf der ersten Kolonne wird dabei durch die Kondensation der Brüden aus der zweiten Kolonne beheizt. Der Wärmeaustausch kann dabei mittels interner (z.B. Heizbündel) oder externer Verdampfer (z.B. Umlaufverdampfer) erfolgen. Parallel hierzu kann noch ein weiterer Verdampfer eingesetzt werden, z.B. zur Nutzung einer weiteren Energiequelle auf niedrigem Temperaturniveau.

Die Brüden der ersten Kolonne werden kondensiert und nach Abtrennung der wasserdampfflüchtigen organischen Phase (der wasserdampfflüchtigen leichtsiedenden Nebenprodukte) durch Phasentrennung wieder auf den Kopf der ersten Kolonne zugeführt. Die Destillatentnahme, d.h. die Entnahme des Wassers, erfolgt mittels eines Entnahmebodens über einen Seitenstrom, der mindestens 4, vorzugsweise 5 bis 15 theoretische Stufen unterhalb des Kopfs der ersten Kolonne und mindestens 8, vorzugsweise 12 bis 25 theoretische Stufen oberhalb des Sumpfs der ersten Kolonne angeordnet ist. Hierbei liegt das Volumenverhältnis von Rücklauf (unterhalb der Entnahmestelle) zu der Entnahme des Wassers vorzugsweise bei mindestens 0,2 vorzugsweise 0,3 bis 0,6.

Das Sumpfprodukt der ersten Kolonne wird oberhalb des Sumpfverdampfers, vorzugsweise zwischen der zweiten und der achten theoretischen Stufe (vom Sumpf der Kolonne) in die zweite Kolonne eingespeist. Der Sumpf der zweiten Kolonne wird mit externen Heizmitteln, z.B. mittels Heizdampf beheizt. Die Beheizung kann mittels interner (z.B. Heizbündel) oder externer Verdampfer (z.B. Umlaufverdampfer) erfolgen. Die Beheizung kann über einen einzelnen Verdampfer oder durch mehrere in Serie verschaltete Verdampfer mit unterschiedlicher Heiztemperatur (z.B. bevorzugt Wasserdampf, aber auch prozessinterne Stoffströme) erfolgen.

Die Brüden aus der zweiten Kolonne, die bei höherem absolutem Druck von 2 bis 20 bar betrieben wird, werden zur Beheizung der ersten Kolonne genutzt. Das Brüdenkondensat aus der zweiten Kolonne wird zum Teil als Rücklauf auf den Kopf der zweiten Kolonne und zum Teil auf den Kopf der ersten Kolonne zur Entfernung der wasserdampfflüchtigen Nebenkomponenten aufgegeben. Das weitgehend von Nebenkomponenten befreite Wasser wird dann als Seitenstrom der ersten Kolonne entnommen.

Das Volumenverhältnis des Anteils des Brüdenkondensats, der als Rücklauf auf die zweite Kolonne aufgegeben wird, und des Anteils des Brüdenkondensats, der auf den Kopf der ersten Kolonne aufgegeben wird, beträgt vorzugsweise mindestens 0,2, besonders bevorzugt 0,3 bis 0,6. Der Verstärkerteil der zweiten Kolonne weist bevorzugt mindestens 15 theoretische Stufen auf.

In einer zweiten Ausführungsform des erfindungsgemäßen Verfahrens wird die erste Kolonne der beiden seriell verschalteten Destillationskolonnen unter höherem absolutem Druck von 2 bis 20 bar betrieben. Die zweite Destillationskolonne (oder im Falle von insgesamt mehr als zwei Destillationskolonnen eine der nachfolgenden Destillationskolonnen) wird also bei niedrigeren Drücken betrieben als die erste Destillationskolonne. Die zu trennende wässrige Aminlösung wird oberhalb des Sumpfverdampfers, vorzugsweise zwischen der zweiten und achten theoretischen Stufe (vom Sumpf der Kolonne) in die erste Kolonne eingespeist. Der Sumpf dieser Kolonne wird mit externen Heizmitteln, z. B. mittels Heizdampf beheizt. Die Beheizung kann mittels interner (z.B. Heizbündel) oder externer Verdampfer (z.B. Umlaufverdampfer) erfolgen. Parallel hierzu kann noch ein weiterer Verdampfer eingesetzt werden, z.B. zur Nutzung einer weiteren Energiequelle.

Die Brüden aus der ersten Kolonne werden durch Kondensation und Wärmeaustausch zur Beheizung der zweiten Kolonne eingesetzt. Der Wärmeaustausch kann dabei mittels interner (z.B. Heizbündel) oder externer Verdampfer (z.B. Umlaufverdampfer) erfolgen. Nach Kondensation der Brüden aus der ersten Kolonne wird ein Teil der Brüden als Rücklauf auf den Kopf der ersten Kolonne aufgegeben, ein Teil auf den Kopf der zweiten Kolonne zur Entfernung der wasserdampfflüchtigen Nebenkomponenten (Rücklaufverhältnis bevorzugt mindestens 0,1, besonders bevorzugt 0,15 bis 0,6). Der Verstärkerteil der ersten Kolonne weist dabei mindestens 15 theoretische Trennstufen auf.

Das Sumpfprodukt der ersten Kolonne wird oberhalb des Sumpfverdampfers, vorzugsweise zwischen der zweiten und der achten theoretischen Stufe (vom Sumpf der Kolonne) in die zweite Kolonne eingespeist. Der Sumpf der zweiten Kolonne wird dabei durch Kondensation der Brüden aus der ersten Kolonne und Wärmeaustausch beheizt. Die Beheizung kann mittels interner (z.B. Heizbündel) oder externer Verdampfer (z.B. Umlaufverdampfer) erfolgen. Zur vollständigen Wasserentfernung bzw. zur Einstellung der angestrebten Endkonzentration an Wasser wird die zweiten Kolonne mit einem zusätzlichen Verdampfer betrieben, der mit Heizdampf betrieben werden kann. Je nach Heizdampfniveau bzw. Druckstufe des eingesetzten Heizdampfes können dazu auch mehrere in Serie verschaltete Verdampfer mit unterschiedlicher Heiztemperatur (z.B. auch prozeßinterne Stoffströme) eingesetzt werden.

Die Brüden aus der zweiten Kolonne werden kondensiert und nach Abtrennung der wasserdampfflüchtigen organischen Phase (der wasserdampfflüchtigen leichtsiedenden Nebenprodukte) durch Phasentrennung wieder auf den Kopf der zweiten Kolonne zurückgeführt. Im oberen Teil der zweiten Kolonne werden die wasserdampfflüchtigen Leichtsieder entfernt und das weitgehend von Nebenkomponenten befreite Wasser als Seitenstrom entnommen. Die Destillatentnahme, d.h. die Entnahme des Wassers, erfolgt mittels eines Entnahmebodens über einen Seitenstrom, der mindestens 4, vorzugsweise 5 bis 15 theoretische Stufen unterhalb des Kopfs der zweiten Kolonne und mindestens 8, vorzugsweise 12 bis 25 theoretische Stufen oberhalb des Sumpfs der zweiten Kolonne angeordnet ist. Hierbei liegt das Volumenverhältnis von Rücklauf (unterhalb der Entnahmestelle) zu Entnahme des Wassers bei mindestens 0,2 vorzugsweise 0,3 bis 0,6.

Das erfindungsgemäße Verfahren wird anhand der folgenden Figuren näher erläutert:

Es zeigen
- Figur 1: eine schematische Darstellung der ersten bevorzugten Ausführungsform des Verfahrens, bei dem die erste Destillationskolonne mit dem geringeren absoluten Druck von 0,1 bis 10 bar betrieben wird.
- Figur 2: eine schematische Darstellung der zweiten bevorzugten Ausführungsform des Verfahrens, bei dem die erste Destillationskolonne mit dem höheren absoluten Druck von 2 bis 20 bar betrieben wird.

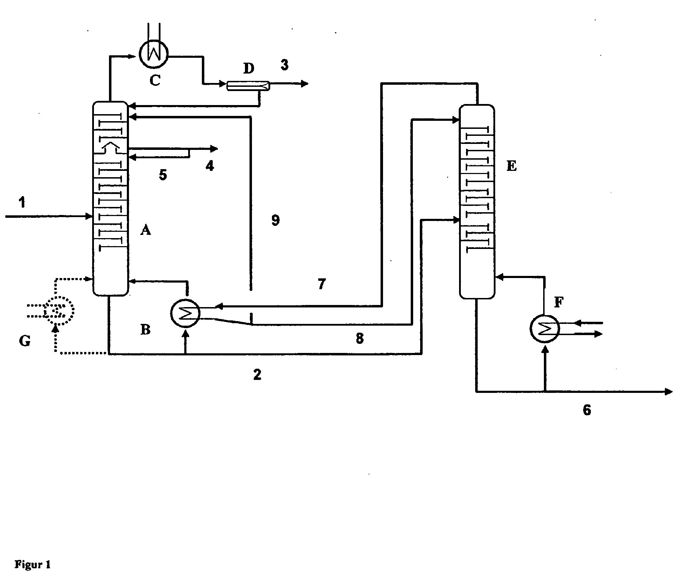

Figur 1 zeigt eine schematische Darstellung der ersten bevorzugten Ausführungsform des Verfahrens, bei dem die erste Destillationskolonne A mit dem geringeren absoluten Druck im Bereich von 0,1 bis 10 bar betrieben wird und die zweite Destillationskolonne E mit dem höheren absoluten Druck im Bereich von 2 bis 20 bar betrieben wird. Die wässrige Aminlösung (Strom 1) wird in die erste Kolonne A eingespeist und aufgetrennt. Die Brüden werden in einem Kondensator C kondensiert, und anschließend in einer Flüssig-Flüssig-Trennapparatur D die Leichtsieder von der wässrigen Phase abgetrennt und als Strom 3 ausgeschleust. Die wässrige Phase wird wieder als Rücklauf zum Kopf der Kolonne A zurückgeführt. Im Seitenstrom wird das von organischen Bestandteilen gereinigte Wasser abgetrennt und teilweise als Strom 4 ausgeschleust und teilweise als Strom 5 als Rücklauf zurückgeführt. Der Sumpf der Kolonne A wird durch einen Verdampfer B und einen optionalen zusätzlichen Wärmeaustauscher bzw. Verdampfer G aufgeheizt.

Der Sumpf der ersten Kolonne A wird teilweise abgezogen und als Strom 2 in die zweite Destillationskolonne E eingespeist.

Die Beheizung des Verdampfers B erfolgt durch die aus der zweiten Kolonne E austretenden Brüden (Strom 7). Die Brüden werden dabei im Verdampfer B kondensiert. Die kondensierten Brüden werden anschließend zum Teil wieder als Rücklauf zum Kopf der zweiten Kolonne E zurückgeführt (Strom 8) und zum Teil auf den Kopf der ersten Kolonne A aufgegeben (Strom 9), um die wasserdampfflüchtigen Nebenkomponenten zu entfernen (Rücklaufverhältnis bevorzugt mindestens 0,1, besonders bevorzugt 0,15 bis 0,6).

Die Beheizung der zweiten Kolonne E erfolgt durch Zufuhr von Fremdenergie, beispielsweise von Heizdampf, in dem Verdampfer F. Das gereinigte Amin wird als Strom 6 aus dem Sumpf der zweiten Kolonne E abgezogen.

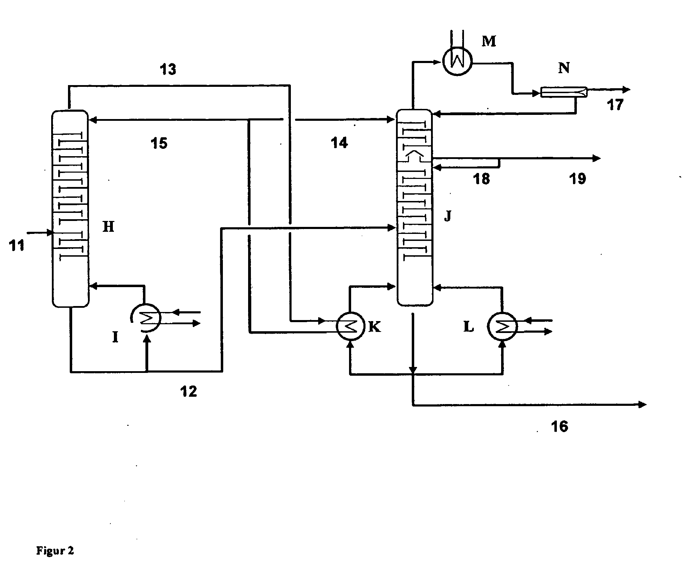

Figur 2 zeigt eine schematische Darstellung der zweiten bevorzugten Ausführungsform des Verfahrens, bei dem die erste Destillationskolonne H mit dem höheren absoluten Druck von 2 bis 20 bar betrieben wird und die zweite Destillationskolonne J mit dem niedrigeren absoluten Druck im Bereich von 0,1 bis 10 bar betrieben wird. Die wässrige Aminlösung (Strom 11) wird in die erste Kolonne H eingespeist und aufgetrennt. Die Beheizung des Sumpfs der ersten Kolonne H erfolgt dabei durch Zufuhr von Fremdenergie, beispielsweise von Heizdampf, in dem Verdampfer I.

Der Sumpf der ersten Kolonne H wird teilweise abgezogen und als Strom 12 in die zweite Destillationskolonne J eingespeist. Die aus der zweiten Kolonne J austretenden Brüden werden in einem Kondensator M kondensiert, und anschließend in einer Flüssig-Flüssig-Trennapparatur N die Leichtsieder von der wässrigen Phase abgetrennt und als Strom 17 ausgeschleust. Die wässrige Phase wird wieder als Rücklauf zum Kopf der zweiten Kolonne J zurückgeführt. Im Seitenstrom wird das von organischen Bestandteilen weitgehend gereinigte Wasser abgetrennt und teilweise als Strom 19 ausgeschleust und teilweise als Strom 18 als Rücklauf zurückgeführt. Der Sumpf der Kolonne J wird durch einen Verdampfer L und einen Verdampfer K aufgeheizt.

Die Beheizung der zweiten Kolonne J durch den zusätzlichen Verdampfer L erfolgt durch Zufuhr von Fremdenergie, beispielsweise von Heizdampf. Die Beheizung der zweiten Kolonne J durch den Verdampfer K erfolgt durch die aus der ersten Kolonne H als Strom 13 austretenden Brüden. Die Brüden werden dabei im Verdampfer K kondensiert. Die kondensierten Brüden werden anschließend zum Teil als Strom 14 wieder als Rücklauf (Rücklaufverhältnis bevorzugt mindestens 0,1, besonders bevorzugt 0,15 bis 0,6) zum Kopf der zweiten Kolonne J zurückgeführt, um die wasserdampfflüchtigen Nebenkomponenten zu entfernen, und zum Teil als Strom 15 auf den Kopf der ersten Kolonne H aufgegeben.

Das gereinigte Amin wird als Strom 16 aus dem Sumpf der zweiten Kolonne J abgezogen.

### Beispiele

### Beispiel 1 (gemäß der Ausführungsform nach Figur 1):

In einer ersten Kolonne A mit 36 Stufen gibt man ein lösungsmittelfreies Reaktionsgemisch aus der DNT-Hydrierung auf die 5. Stufe oberhalb des Sumpfes auf. Es handelt sich bei dem Gemisch um eine ca. 57 gew.-%ige Lösung eines Diamingemischs bestehend im wesentlichen aus 77,2 Gew.-% 2,4-Diaminotoluol, 19,3 Gew.-% 2,6-Diaminotoluol und 3,5 Gew.-% anderen Diaminotoluol-Isomeren. Die Lösung weist einen Gehalt an wasserdampfflüchtigen organischen Nebenprodukten von 0,3 Gew.-% auf. Der Wassergehalt der Lösung liegt entsprechend bei ca. 42,7 Gew.-%.

Diese erste Kolonne A wird mit einem absoluten Kopfdruck von 0,6 bar betrieben. Im Sumpf der Kolonne A stellt sich ein Wassergehalt von 30 Gew.-% bei ca. 93 °C ein. Die Beheizung erfolgt mit den Brüden der zweiten Kolonne E. Die Kolonne E wird mit 3 bar absolutem Druck betrieben, die Brüden weisen entsprechend eine Temperatur von 134 °C auf. Am Kopf der ersten Kolonne A werden die Leichtsieder als Strom 3 aus dem Zulauf nahezu quantitativ abgetrennt, das kondensierte Wasser auf die oberste Stufe zurückgegeben. 10 Stufen unterhalb des Kopfes wird das Wasser als Seitenstrom abgenommen, teilweise als Strom 4 ausgeschleust und teilweise als Strom 5 als Rücklauf zurückgeführt. Um einem Restgehalt von ca. 100 ppm im destillierten Wasser zu erreichen, wird an der Seitenentnahme ein Rücklaufverhältnis von 0,3 gefahren.

Das Sumpfprodukt der ersten Kolonne (Strom 2) wird 5 Stufen oberhalb des Sumpfes in die zweite Kolonne E eingespeist. Diese hat insgesamt 25 Stufen. Im Sumpf der Kolonne E wird ein Wasser-Restgehalt von 3 Gew.-% bei ca. 180 °C erreicht. Das Kopfprodukt der Kolonne E (Brüdenstrom 7) wird im Verdampfer B der ersten Kolonne A kondensiert. 3 Teile des Brüdenkondensats werden als Rücklauf 8 auf die zweite Kolonne E zurückgeführt, 10 Teile des Brüdenkondensats werden als Strom 9 auf die oberste Stufe der ersten Kolonne A geführt.

### Beispiel 2 (gemäß der Ausführungsform nach Figur 2):

In einer ersten Kolonne H mit 25 theoretischen Stufen gibt man das in Beispiel 1 bereits beschriebene Reaktionsgemisch aus der DNT-Hydrierung auf die 5. Stufe oberhalb des Sumpfes auf.

Die Kolonne H wird mit einem absoluten Kopfdruck von 3 bar betrieben; im Sumpf der Kolonne H stellt sich ein Wassergehalt von 30 Gew.-% bei ca. 140°C ein. Die Beheizung des Sumpfs im Verdampfer I erfolgt mittels Heizdampf von 160°C. Der Brüdenstrom 13 der ersten Kolonne H wird in dem Verdampfer K kondensiert, in dem gleichzeitig der Sumpf der zweiten Kolonne J erhitzt und verdampft wird. 3 Teile des Brüdenkondensats werden als Rücklauf (Strom 15) auf den Kopf der ersten Kolonne H zurückgeführt, 10 Teile des Brüdenkondensats werden als Strom 14 auf den Kopf der zweiten Kolonne J geführt.

Das Sumpfprodukt der ersten Kolonne H (Strom 12) wird 5 Stufen oberhalb des Sumpfes in die zweite Kolonne J eingespeist. Die Kolonne J hat insgesamt 36 Stufen und wird mit einem absoluten Kopfdruck von 3 bar betrieben. Im Sumpf der Kolonne J wird ein Wasser-Restgehalt von 3 Gew.-% bei ca. 120°C erreicht. Am Kopf der zweiten Kolonne J werden die Brüden im Kondensator M kondensiert und die Leichtsieder nahezu quantitativ in der Flüssig-Flüssig-Trennapparaturr N abgetrennt und als Strom 17 ausgeschleust. Das kondensierte Wasser wird auf die oberste Stufe zurückgegeben. 10 Stufen unterhalb des Kopfes wird das Wasser als Seitenstrom abgenommen, teilweise als Strom 19 ausgeschleust und teilweise als Strom 18 als Rücklauf zurückgeführt. Um einem Restgehalt von ca. 100 ppm im destillierten Wasser zu erreichen, wird an der Seitenentnahme ein Rücklaufverhältnis von 0,3 gefahren.

Im Vergleich zu einstufigen Verdampfungsanlagen nach dem Stand der Technik (z.B. gemäß EP 0 236 839 B1) lassen sich hiermit 30 bis 50 % des Energieverbrauchs einsparen.

## Patentansprüche

1. Verfahren zur destillativen Trennung von wässrigen Aminlösungen, die bei der Hydrierung von Nitroaromaten anfallen, **dadurch gekennzeichnet, dass**
a) die Destillation in mindestens zwei in Serie verschalteten Destillationskolonnen durchgeführt wird, die mit unterschiedlichen Drücken betrieben werden, wobei wenigstens eine Destillationskolonne bei absoluten Drücken am Kopf von 2 bis 20 bar betrieben wird und mindestens eine Destillationskolonne bei absoluten Drücken am Kopf von 0,1 bis 10 bar betrieben wird, und
b) die aus der mit höheren Drücken betriebenen Destillationskolonne austretenden Brüden zumindest teilweise kondensiert werden und die dabei freiwerdende Wärme zur Beheizung des Sumpfs der bei den niedrigeren Drücken betriebenen Destillationskolonne verwendet wird, und
c) die Aminlösung in die erste der mindestens zwei in Serie verschalteten Destillationskolonnen geführt wird, und der Sumpf der ersten Destillationskolonne teilweise abgezogen und in die zweite Destillationskolonne eingespeist wird, und
d) das gereinigte Amin als Sumpfprodukt der letzten Destillationskolonne abgezogen wird.

2. Verfahren nach Anspruch 1, **dadurch gekennzeichnet, dass** zusätzlich
e) die Brüden aus der mit den geringeren Drücken betriebenen Destillationskolonne kondensiert werden, die wasserdampfflüchtigen Leichtsieder durch Phasentrennung daraus abgetrennt werden, und die verbleibenden wässrigen kondensierten Brüden als Rücklauf in die mit den geringeren Drücken betrieben Destillationskolonne zurückgeführt werden, und
f) das Brüdenkondensat aus der mit den höheren Drücken betriebenen Destillationskolonne teilweise auf den Kopf der mit den niedrigeren Drücken betriebenen Destillationskolonne aufgegeben wird und teilweise als Rücklauf auf den Kopf der mit den höheren Drücken betriebenen Kolonne aufgegeben wird, und
g) Wasser als Seitenstrom aus der mit den niedrigeren Drücken betriebenen Destillationskolonne abgezogen wird.

3. Verfahren nach einem der Ansprüche 1 oder 2, **dadurch gekennzeichnet, dass** als wässrige Aminlösungen solche von aromatischen Diaminen eingesetzt werden.

4. Verfahren nach einem der Ansprüche 1 oder 2, **dadurch gekennzeichnet, dass** als wässrige Aminlösungen solche von Diaminotoluolen eingesetzt werden.

5. Verfahren nach einem der Ansprüche 1 bis 4, **dadurch gekennzeichnet, dass** die erste Destillationskolonne mit den niedrigen absoluten Drücken im Bereich von 0,1 bis 10 bar betrieben wird.

6. Verfahren nach einem der Ansprüche 1 bis 4, **dadurch gekennzeichnet, dass** die erste Destillationskolonne mit den höheren absoluten Drücken im Bereich von 2 bis 20 bar betrieben wird.

7. Verfahren nach einem der Ansprüche 1 bis 6, **dadurch gekennzeichnet, dass** die mindestens zwei in Serie geschalteten Destillationskolonnen jeweils über 12 bis 50 theoretische Stufen verfügen.

8. Verfahren nach Anspruch 7, **dadurch gekennzeichnet, dass** die Entnahme des Wassers im Seitenstrom in Schritt g) aus der mit niedrigeren Drücken betriebenen Destillationskolonne mindestens vier theoretische Stufen unterhalb des Kopfes und mindestens 8 theoretische Stufen oberhalb des Sumpfs erfolgt.

9. Verfahren nach einem der Ansprüche 2 bis 8, **dadurch gekennzeichnet, dass** das Verhältnis von Rücklauf zu Entnahme an der Entnahmestelle für das Wasser in Schritt g) bei mindestens 0,2 liegt.

## Claims

1. Process for the distillative separation of aqueous amine solutions that occur in the hydrogenation of nitroaromatic compounds, **characterised in that**
a) the distillation is carried out in at least two distillation columns connected in series that are operated at different pressures, in which at least one distillation column is operated at absolute pressures at the column head of 2 to 20 bar and at least one distillation column is operated at absolute pressures at the column head of 0.1 to 10 bar, and
b) the vapours leaving the distillation column operating at higher pressures are at least partially condensed and the heat that is thereby released is used to heat the bottom of the distillation column operating at the lower pressures, and
c) the amine solution is fed into the first of the at least two distillation columns connected in series, and the bottom product of the first distillation column is at least partially removed and fed into the second distillation column, and
d) the purified amine is removed as bottom product from the last distillation column.

2. Process according to claim 1, **characterised in that** in addition
e) the vapours from the distillation column operating at the lower pressures are condensed, the steam-volatile low-boiling compounds are separated therefrom by phase separation, and the remaining aqueous condensed vapours are recycled as reflux stream to the distillation column operating at the lower pressures, and
f) the vapour condensate from the distillation column operating at the higher pressures is fed in part to the head of the distillation column operating at the lower pressures and in part as reflux stream to the head of the column operating at the higher pressures, and
g) water is removed as side stream from the distillation column operating at the lower pressures.

3. Process according to one of claims 1 or 2, **characterised in that** aqueous solutions of aromatic diamines are used as aqueous amine solutions.

4. Process according to one of claims 1 or 2, **characterised in that** aqueous solutions of diaminotoluenes are used as aqueous amine solutions.

5. Process according to one of claims 1 to 4, **characterised in that** the first distillation column is operated at low absolute pressures in the range from 0.1 to 10 bar.

6. Process according to one of claims 1 to 4, **characterised in that** the first distillation column is operated at higher absolute pressures in the range from 2 to 20 bar.

7. Process according to one of claims 1 to 6, **characterised in that** the at least two distillation columns connected in series include in each case 12 to 50 theoretical plates.

8. Process according to claim 7, **characterised in that** the removal of the water in the side stream in step g) from the distillation column operating at lower pressures takes place at least four theoretical plates below the head of the column and at least eight theoretical plates above the bottom of the column.

9. Process according to one of claims 2 to 8, **characterised in that** the ratio of reflux to removal at the removal site for the water in step g) is at least 0.2.

## Revendications

1. Procédé de séparation par distillation de solutions aqueuses d'amine, qui se présentent lors de l'hydrogénation de composés nitroaromatiques, **caractérisé en ce que**
a) la distillation est réalisée dans au moins deux colonnes de distillation connectées en série, qui sont opérées à des pressions différentes, au moins une colonne de distillation étant opérée à des pressions absolues en tête de colonne comprises entre 2 et 20 bar et au moins une colonne de distillation étant opérée à des pressions absolues en tête de colonne comprises entre 0,1 et 10 bar et
b) les vapeurs sortant de la colonne de distillation opérée avec les pressions plus élevées sont au moins partiellement condensées et la chaleur ainsi dégagée est utilisée pour chauffer le fond de la colonne de distillation opérée avec les pressions plus basses et
c) la solution d'amine est acheminée dans la première des au moins deux colonnes de distillation connectées en série et le fond de la première colonne de distillation est partiellement aspiré et alimenté dans la deuxième colonne de distillation et
d) l'amine purifiée est aspirée comme produit de fond de la dernière colonne de distillation.

2. Procédé selon la revendication 1, **caractérisé en ce qu'**en plus
e) les vapeurs provenant de la colonne de distillation opérée avec les pressions plus basses sont condensées, les fractions de bas point d'ébullition entraînables à la vapeur d'eau sont séparées de celles-ci par séparation de phases, et les vapeurs aqueuses condensées restantes sont recyclées comme courant de retour à la colonne de distillation opérée avec les pressions plus basses et
f) le produit de condensation des vapeurs provenant de la colonne de distillation opérée avec les pressions plus élevées est alimenté partiellement à la tête de la colonne de distillation opérée avec les pressions plus basses et partiellement comme courant de retour à la tête de la colonne opérée avec les pressions plus élevées et
g) de l'eau est aspirée comme courant latéral de la colonne de distillation opérée avec les pressions plus basses.

3. Procédé selon une des revendications 1 ou 2, **caractérisé en ce que** comme solutions aqueuses d'amine, on utilise celles de diamines aromatiques.

4. Procédé selon une des revendications 1 ou 2, **caractérisé en ce que** l'on utilise comme solutions aqueuses d'amine, celles de diaminotoluènes.

5. Procédé selon une des revendications 1 à 4, **caractérisé en ce que** la première colonne de distillation est opérée avec des pressions absolues basses comprises dans l'intervalle de 0,1 à 10 bar.

6. Procédé selon une des revendications 1 à 4, **caractérisé en ce que** la première colonne de distillation est opérée avec des pressions absolues plus élevées comprises dans l'intervalle de 2 à 20 bar.

7. Procédé selon une des revendications 1 à 6, **caractérisé en ce que** les au moins deux colonnes de distillation connectées en série disposent chacune de 12 à 50 plateaux théoriques.

8. Procédé selon la revendication 7, **caractérisé en que** le soutirage de l'eau dans le courant latéral dans l'étape g) à partir de la colonne de distillation opérée avec les pressions plus basses a lieu au moins quatre plateaux théoriques au-dessous de la tête et au moins 8 plateaux théoriques au-dessus du fond de la colonne.

9. Procédé selon au moins une des revendications 2 à 8, **caractérisé en ce que** le rapport entre le retour et le soutirage au point de soutirage de l'eau dans l'étape g) est d'au moins 0,2.
